# EUROPEAN PATENT APPLICATION

(11) **EP 4 732 880 A1**
(43) Date of publication of application: **29.04.2026**
(21) Application number: 24825927.7
(22) Date of filing: 19.06.2024
(51) Int. Cl.: A61M 25/10

(54) **BALLOON CATHETER**

(30) Priority: 22.06.2023 JP 2023102816
(71) Applicant: KANEKA CORPORATION, Osaka 530-8288 (JP)
(72) Inventor: MATSUFUJI, Kazuki, Settsu-shi, Osaka 566-0072 (JP); KOJIMA, Masahiro, Settsu-shi, Osaka 566-0072 (JP); HAMABUCHI, Takahisa, Osaka-shi, Osaka 530-8288 (JP)
(74) Representative: Plasseraud IP
(86) International application number: PCT/JP2024/022197
(87) International publication number: WO 2024/262526

(57) **Abstract**

Provided is a balloon catheter that can apply pressure precisely to the target site. A balloon catheter (1) including: a shaft (10) extending in a longitudinal direction from a proximal side to a distal side; a first balloon (21) disposed at a distal part of the shaft (10); and a balloon group (30) including a plurality of second balloons (22) disposed outside the first balloon (21) and arranged in a circumferential direction of the first balloon (21), wherein in an inflated state of the first balloon (21) and the balloon group (30), a length (L1) of the first balloon (21) from a distal end (21d) thereof to a proximal end (21p) thereof in the longitudinal direction is shorter than a length (L2) of at least one of the plurality of second balloons (22) from a distal end (22d) thereof to a proximal end (22p) thereof in the longitudinal direction.

## Description

### TECHNICAL FIELD

The present invention relates to a balloon catheter.

### BACKGROUND ART

Diseases such as angina pectoris and myocardial infarction are caused by the formation of a stenotic part hardened by calcification and other factors in inner walls of blood vessels. One of the treatments for these diseases is angioplasty, in which a balloon catheter is used to dilate the stenotic part. The angioplasty is a minimally invasive therapy that does not require an open chest procedure like bypass surgery and is widely used.

There is a disorder called aortic valve stenosis in which an aortic valve is hardened by calcification and other factors and has difficulty in opening so that the flow of blood is blocked. As a treatment for such aortic valve stenosis, a method including implanting a bioprosthetic valve (prosthetic valve) by either of a surgical open chest procedure and a catheter to replace a hardened aortic valve with the bioprosthetic valve is sometimes employed.

Such an implanted bioprosthetic valve degrades over time owing to calcification, wear, or the like. When an implanted bioprosthetic valve degrades, the bioprosthetic valve needs to be replaced. A procedure has been studied for such replacement of a bioprosthetic valve. In the procedure, a high pressure is applied to an implanted bioprosthetic valve by using a balloon catheter having a braid or a plurality of balloon catheters to deform or rupture the bioprosthetic valve, and, in a state where a lumen of the valve is widened, a new bioprosthetic valve is implanted into the deformed or ruptured bioprosthetic valve through transcatheter aortic valve replacement or the like.

As catheters to be used for dilation of a hardened stenosis site or implantation of a bioprosthetic valve and capable of inflating a balloon at a high pressure, for example, Patent document 1 discloses a balloon catheter having a plurality of balloon members including an inner balloon member and a plurality of outer balloon members disposed to enclose the outer surface of the inner balloon member, and Patent document 2 discloses a device having a perfusion balloon having an internal passage and a balloon disposed in the internal passage of the perfusion balloon.

### RELATED ART DOCUMENT

### PATENT DOCUMENT

Patent document 1: US 2012/0209375A1
Patent document 2: JP 2018-536474 A

### SUMMARY OF THE INVENTION

### PROBLEMS TO BE SOLVED BY THE INVENTION

However, conventional balloons such as those described in Patent documents 1 and 2 sometimes have difficulty in applying pressure precisely to a site to which pressure is desired to be applied, at the time of dilating a stenosis site or deforming or rupturing an implanted bioprosthetic valve. Thus, problems arise in that: dilation of the stenosis site is insufficient; the bioprosthetic valve cannot be sufficiently deformed or ruptured; and it takes time to dilate the stenosis site or to deform or rupture the bioprosthetic valve. In particular, if the bioprosthetic valve has not been sufficiently deformed or ruptured, the lumen of the aortic valve narrows, and the flow rate of blood decreases.

In view of the above circumstances, the objective of the present invention is to provide a balloon catheter that can apply pressure precisely to the target site.

### MEANS FOR SOLVING THE PROBLEMS

A balloon catheter in accordance with an embodiment of the present invention that can solve the above problem is as follows.
[1] A balloon catheter including: a shaft extending in a longitudinal direction from a proximal side to a distal side; a first balloon disposed at a distal part of the shaft; and a balloon group including a plurality of second balloons disposed outside the first balloon and arranged in a circumferential direction of the first balloon, wherein in an inflated state of the first balloon and the balloon group, a length of the first balloon from a distal end thereof to a proximal end thereof in the longitudinal direction is shorter than a length of at least one of the plurality of second balloons from a distal end thereof to a proximal end thereof in the longitudinal direction.
[2] The balloon catheter according to [1], wherein the first balloon and the at least one of the plurality of second balloons each have a straight tubular part, a proximal tapered part located proximal to the straight tubular part, and a distal tapered part located distal to the straight tubular part, and in the inflated state of the first balloon and the balloon group, a proximal end of the proximal tapered part of the first balloon is located distal to a proximal end of the proximal tapered part of the at least one of the plurality of second balloons, and a distal end of the distal tapered part of the first balloon is located proximal to a distal end of the distal tapered part of the at least one of the plurality of second balloons.
[3] The balloon catheter according to [2], wherein in the inflated state of the first balloon and the balloon group, an outer diameter of the balloon group at a midpoint of a length of the balloon group in the longitudinal direction is larger than an outer diameter of the balloon group at a distal end of the straight tubular part of the at least one of the plurality of second balloons and an outer diameter of the balloon group at a proximal end of the straight tubular part of the at least one of the plurality of second balloons.
[4] The balloon catheter according to any one of the above [1] to [3], wherein in the inflated state of the first balloon and the balloon group, at least one pair of adjacent second balloons among the plurality of second balloons constituting the balloon group are in contact with each other.
[5] The balloon catheter according to any one of the above [1] to [4], wherein in the inflated state of the first balloon and the balloon group, the balloon group is in contact with an outer peripheral surface of the first balloon.
[6] The balloon catheter according to any one of the above [1] to [5], wherein the shaft has a first inflation lumen and a second inflation lumen extending in the longitudinal direction, the first inflation lumen is connected to the first balloon, and the second inflation lumen is connected to the at least one of the plurality of second balloons constituting the balloon group.
[7] The balloon catheter according to any one of the above [1] to [6], wherein the shaft has a guide wire lumen extending in the longitudinal direction and through which a guide wire is inserted, the balloon catheter further includes a guide wire tube having a lumen in communication with the guide wire lumen, and the guide wire tube is disposed in a lumen of the first balloon.
[8] The balloon catheter according to any one of the above [1] to [7], wherein the balloon catheter is used for dilation of an aortic valve, deformation of a bioprosthetic valve implanted in a heart, or rupture of the bioprosthetic valve.

### EFFECTS OF THE INVENTION

In the above balloon catheter, in the inflated state of the first balloon and the balloon group, the length of the first balloon in the longitudinal direction is shorter than the length of the at least one second balloon in the longitudinal direction, whereby a portion at which the first balloon is present is easily inflated to a larger extent than a portion at which the first balloon is not present. As a result, at the portion of the balloon catheter at which the first balloon is present, pressure is easily applied, whereby pressure can be applied precisely to the target site.

### BRIEF DESCRIPTION OF THE DRAWINGS

[FIG. 1] FIG. 1 is a side view of a balloon catheter in accordance with one embodiment of the present invention.
[FIG. 2] FIG. 2 is a side view of a balloon of the balloon catheter shown in FIG. 1.
[FIG. 3] FIG. 3 is a III-III cross-sectional view of the balloon catheter shown in FIG. 1.
[FIG. 4] FIG. 4 is a IV-IV cross-sectional view of the balloon catheter shown in FIG. 1.
[FIG. 5] FIG. 5 is a V-V cross-sectional view of the balloon catheter shown in FIG. 1.

### MODE FOR CARRYING OUT THE INVENTION

Hereinafter, the present invention will be described based on the following embodiments, however, the present invention is not limited by the following embodiments and can be altered in design within a scope in compliance with the intent described above and below, and all the changes are to be encompassed within a technical scope of the present invention. Note that, in each drawing, hatching, reference signs for components, and the like may be omitted for convenience of description, and in such a case, the specification and other drawings are to be referred to. Furthermore, since the dimensions of the various components in the drawings are provided for the purpose of facilitating the understanding of the feature of the present invention, the dimensions may differ from the actual dimensions in some cases.

A balloon catheter in accordance with an embodiment of the present invention is a balloon catheter including: a shaft extending in a longitudinal direction from a proximal side to a distal side; a first balloon disposed at a distal part of the shaft; and a balloon group including a plurality of second balloons disposed outside the first balloon and arranged in a circumferential direction of the first balloon, wherein, in an inflated state of the first balloon and the balloon group, a length of the first balloon from a distal end thereof to a proximal end thereof in the longitudinal direction is shorter than a length of at least one of the plurality of second balloons from a distal end thereof to a proximal end thereof in the longitudinal direction.

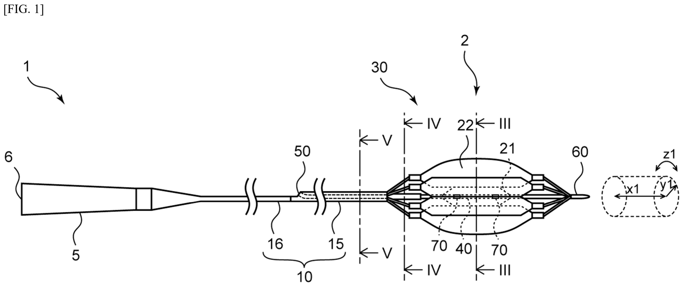

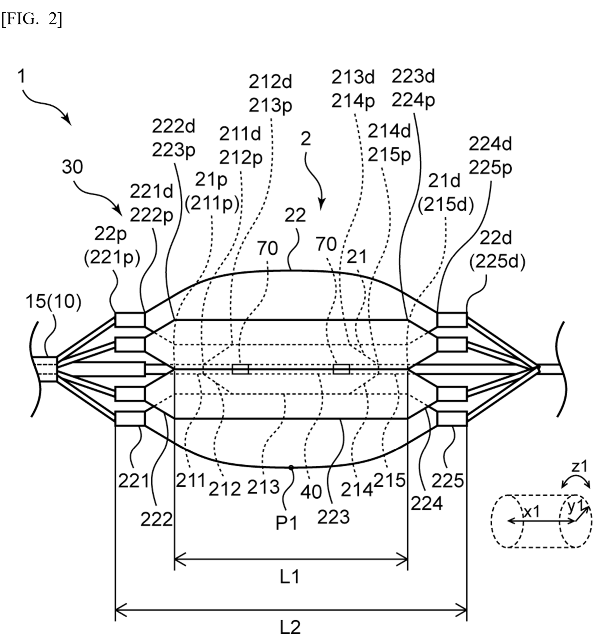

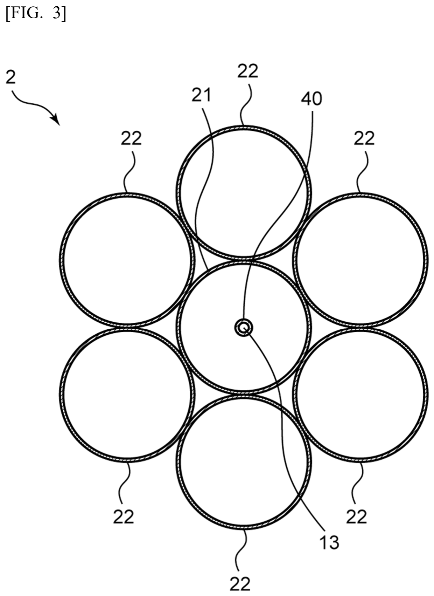

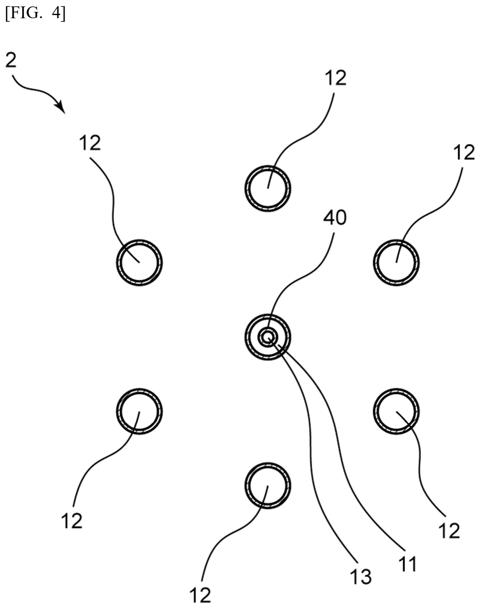

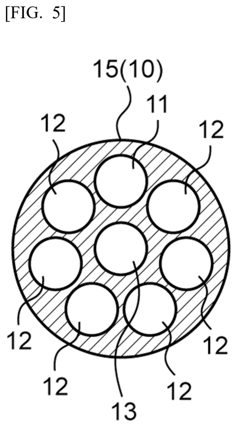

Hereinafter, a balloon catheter in accordance with embodiments of the present invention will be described, referring to FIG. 1 to FIG. 5. FIG. 1 is a side view of a balloon catheter in accordance with one embodiment of the present invention, and FIG. 2 is a side view of a balloon of the balloon catheter shown in FIG. 1. FIG. 3 is a III-III cross-sectional view of the balloon catheter shown in FIG. 1, illustrating a cross-sectional view perpendicular to the longitudinal direction at a portion where the first balloon and the balloon group are present. FIG. 4 is a IV-IV cross-sectional view of the balloon catheter shown in FIG. 1, illustrating a cross-sectional view perpendicular to the longitudinal direction at a portion where the first balloon and the balloon group are connected to the shaft. FIG. 5 is a V-V cross-sectional view of the balloon catheter shown in FIG. 1, illustrating a cross-sectional view perpendicular to the longitudinal direction of the shaft.

As shown in FIG. 1 and FIG. 2, a balloon catheter 1 includes a shaft 10 extending in a longitudinal direction from a proximal side to a distal side, a first balloon 21 disposed at a distal part of the shaft 10 and a balloon group 30 including a plurality of second balloons 22 disposed outside the first balloon 21 and arranged in a circumferential direction of the first balloon 21. In this specification, a balloon of the balloon catheter 1, which includes the first balloon 21 and the plurality of second balloons 22 constituting the balloon group 30, may be referred to simply as "balloon 2".

The shaft 10 has a longitudinal axis direction x1, a radial direction y1 connecting the figure center of the outer edge of the shaft 10 and a point on the outer edge in a cross-section perpendicular to the longitudinal axis direction x1, and a circumferential direction z1 along the outer edge of the shaft 10 in a cross-section perpendicular to the longitudinal axis direction x1. In this specification, the direction of the user's hand side in the longitudinal axis direction x1 is referred to as a proximal side, and the direction opposite the proximal side, i.e., the direction of the treatment target side, is referred to as a distal side.

The components and parts other than the shaft 10 have their own longitudinal axis direction, radial direction, and circumferential direction, which may be the same as or different from the longitudinal axis direction x1, radial direction y1, and circumferential direction z1 of the shaft 10. In this specification, for the sake of ease of understanding, all components and parts are described as having the same longitudinal axis direction, radial direction, and circumferential direction as the longitudinal axis direction x1, radial direction y1, and circumferential direction z1 of the shaft 10.

The first balloon 21 and the second balloons 22 are connected to the distal part of the shaft 10. Introduction of a fluid through a lumen of the shaft 10 enables inflation of the first balloon 21 and the second balloons 22. Meanwhile, discharge of the fluid enables deflation of the first balloon 21 and the second balloons 22. In order to control inflation and deflation of the first balloon 21 and the second balloons 22, an indeflator (balloon pressurizer) may be used to introduce or discharge the fluid. As the fluid, for example, a physiological saline or a liquid mixture of a contrast medium and a physiological saline is used. The fluid may be a pressurized fluid obtained through pressurization by a pump or the like.

The number of the first balloons 21 may be two or more but is preferably one. That is, the balloon catheter 1 preferably has one first balloon 21 and a plurality of second balloons 22. By the number of the first balloons 21 being one, the first balloon 21 becomes less likely to move on the inner side of the balloon group 30 in an inflated state of the first balloon 21 and the balloon group 30. As a result, the first balloon 21 easily suppresses inflation of the plurality of second balloons 22 constituting the balloon group 30, and the hardness of the balloon 2 is increased, whereby expansive force of the balloon 2 can be easily increased.

Examples of a material forming the first balloon 21 include: polyamide resins such as nylon 11 and nylon 12; polyester resins such as polyethylene terephthalate and polybutylene terephthalate; polyurethane resins; and thermoplastic elastomers such as polyether block amide copolymers.

As shown in FIG. 1 and FIG. 2, the balloon group 30 includes the plurality of second balloons 22. The plurality of second balloons 22 constituting the balloon group 30 are disposed outside the first balloon 21 and arranged in the circumferential direction of the first balloon 21. That is, the plurality of second balloons 22 constituting the balloon group 30 are disposed along an outer periphery of the first balloon 21. The balloon 2 has the first balloon 21 and the balloon group 30 including the plurality of second balloons 22, and the plurality of second balloons 22 constituting the balloon group 30 are disposed outside the first balloon 21 and arranged in the circumferential direction of the first balloon 21. Thus, the balloon group 30 suppresses outward inflation of the first balloon 21, and the first balloon 21 suppresses inward inflation of the second balloons 22 constituting the balloon group 30. As a result, the first balloon 21 and the balloon group 30 mutually suppress respective inflations. Consequently, the withstanding pressure of the balloon 2 is made high, and the hardness of the balloon 2 is increased, whereby the expansive force of the balloon 2 can be improved. Furthermore, since the first balloon 21 and the balloon group 30 mutually suppress respective inflations, the balloon 2 becomes less likely to be inflated. Consequently, the balloon 2 is inhibited from being excessively inflated even when a high pressure is applied to the balloon 2. Thus, the balloon 2 is prevented from being inflated to an outer diameter larger than an intended outer diameter, and damage to a lumen inside a living organism such as the aortic valve is decreased, whereby safety can be improved.

The number of the second balloons 22 constituting the balloon group 30 is preferably three or more, more preferably four or more, and further preferably five or more. By setting the lower limit value of the number of the second balloons 22 constituting the balloon group 30 to the above range, the balloon group 30 easily encloses the outer periphery of the first balloon 21 and easily suppresses inflation of the first balloon 21. As a result, when a fluid is introduced into both the first balloon 21 and the balloon group 30 to inflate the balloon 2, the first balloon 21 is less likely to be inflated, and the hardness of the first balloon 21 is increased, whereby the expansive force of the balloon 2 can be increased. Meanwhile, the number of the second balloons 22 constituting the balloon group 30 is preferably 20 or less, more preferably 12 or less, further preferably 10 or less, and particularly preferably eight or less. By setting the upper limit value of the number of the second balloons 22 constituting the balloon group 30 to the above range, when the balloon 2 is inflated, the second balloons 22 constituting the balloon group 30 are less likely to move in the circumferential direction z1, and the balloon group 30 easily suppresses inflation of the first balloon 21.

As materials forming the second balloons 22, the materials presented as examples of the material forming the first balloon 21 are usable. The materials forming the second balloons 22 may be identical to or different from the material forming the first balloon 21.

The materials forming the plurality of respective second balloons 22 constituting the balloon group 30 may be different from one another but are preferably identical to one another. That is, the balloon group 30 preferably includes a plurality of second balloons 22 made of materials identical to one another. By the materials forming the plurality of respective second balloons 22 being identical to one another, the extent of inflation, the hardness, and the like of the balloon group 30 can be made approximately equal in the circumferential direction z1 of the balloon group 30.

In an inflated state of the balloon group 30, the maximum outer diameters of the plurality of respective second balloons 22 constituting the balloon group 30 may be different from one another but are preferably equal to one another. The maximum outer diameters of the plurality of respective second balloons 22 constituting the balloon group 30 being equal to one another means that the maximum outer diameters of the plurality of respective second balloons 22 constituting the balloon group 30 are approximately equal to one another, and specifically means that the maximum outer diameter of one of the second balloons 22 is 90% or more and 110% or less of the maximum outer diameters of all the other second balloons 22. By the maximum outer diameters of the plurality of respective second balloons 22 constituting the balloon group 30 being equal to one another in the inflated state of the balloon group 30, the inflation timings for all of the respective second balloons 22 constituting the balloon group 30 are easily set to coincide with one another, whereby inflation of the balloon 2 is easily controlled. The inflated state of the first balloon 21 means a state where a fluid is introduced into a lumen of the first balloon 21 so that the first balloon 21 is inflated. The inflated state of the balloon group 30 means a state where a fluid is introduced into each of lumens of the plurality of second balloons 22 constituting the balloon group 30 so that all of the second balloons 22 constituting the balloon group 30 are inflated.

In the inflated state of the first balloon 21 and the balloon group 30, the maximum outer diameter of the first balloon 21 may be different from the maximum outer diameters of the plurality of respective second balloons 22 constituting the balloon group 30 but is preferably equal to these maximum outer diameters. The maximum outer diameter of the first balloon 21 being equal to the maximum outer diameters of the plurality of respective second balloons 22 constituting the balloon group 30 means that the maximum outer diameter of the first balloon 21 and the maximum outer diameters of the plurality of respective second balloons 22 constituting the balloon group 30 are approximately equal to each other, and specifically means that the maximum outer diameter of the first balloon 21 is 90% or more and 110% or less of the average value of the maximum outer diameters of the plurality of respective second balloons 22. By the maximum outer diameter of the first balloon 21 being equal to the maximum outer diameters of the plurality of respective second balloons 22 constituting the balloon group 30 in the inflated state of the balloon 2, balance is easily attained between a force of inflation of the first balloon 21 and forces of inflation of the second balloons 22 to suppress the inflation of the first balloon 21. As a result, the hardnesses of the first balloon 21 and the balloon group 30 are increased, and the expansive force of the balloon 2 can be easily increased.

In the inflated state of the balloon group 30, lengths L2 of the plurality of respective second balloons 22 constituting the balloon group 30 from distal ends 22d thereof to proximal ends 22p thereof in the longitudinal direction x1 of the second balloons 22 may be different from one another but are preferably equal to one another. The lengths L2 of the plurality of respective second balloons 22 constituting the balloon group 30 from the distal ends 22d thereof to the proximal ends 22p thereof in the longitudinal direction x1 of the second balloons 22 being equal to one another means that the lengths L2 of the plurality of respective second balloons 22 constituting the balloon group 30 in the longitudinal direction x1 are approximately equal to one another, and specifically means that the length L2 of one of the second balloons 22 in the longitudinal direction x1 is 90% or more and 110% or less of the lengths L2 of all the other second balloons 22 in the longitudinal direction x1. By the lengths L2 of the plurality of respective second balloons 22 constituting the balloon group 30 in the longitudinal direction x1 being equal to one another in the inflated state of the balloon group 30, the inflation timings for all of the respective second balloons 22 are easily set to coincide with one another, whereby inflation of the balloon 2 can be easily controlled.

In the inflated state of the first balloon 21 and the balloon group 30, a length L1 of the first balloon 21 from a distal end 21d thereof to a proximal end 21p thereof in the longitudinal direction x1 is shorter than the length L2 of every second balloon 22 from the distal end 22d thereof to the proximal end 22p thereof in the longitudinal direction x1. Since the length L1 of the first balloon 21 is shorter than the length L2 of the second balloon 22, the first balloon 21 is restrained by each of the plurality of second balloons 22, whereby the first balloon 21 becomes less likely to be displaced. A portion at which the first balloon 21 is present is easily inflated to a larger extent than a portion at which the first balloon 21 is not present. Consequently, at the portion at which the first balloon 21 is present, pressure is easily applied, whereby pressure can be applied precisely to the target site. Meanwhile, at the portion at which the first balloon 21 is not present, the balloon 2 is less likely to be inflated to a large extent, whereby pressure can be made less likely to be applied. Consequently, a load is less likely to be applied to a site other than the target site, whereby the minimal invasiveness of the balloon catheter 1 can be improved.

In the inflated state of the first balloon 21 and the balloon group 30, the length L1 of the first balloon 21 from the distal end 21d thereof to the proximal end 21p thereof in the longitudinal direction x1 is preferably 95% or less, more preferably 90% or less, and further preferably 85% or less of the length L2 of the second balloon 22 from the distal end 22d thereof to the proximal end 22p thereof in the longitudinal direction x1. By setting the upper limit value of the ratio of the length L1 of the first balloon 21 to the length L2 of the second balloon 22 to the above range, the balloon catheter 1 can be set to easily apply a high pressure precisely to the target site. Meanwhile, in the inflated state of the first balloon 21 and the balloon group 30, the length L1 of the first balloon 21 from the distal end 21d thereof to the proximal end 21p thereof in the longitudinal direction x1 is preferably 20% or more, more preferably 25% or more, and further preferably 30% or more of the length L2 of the second balloon 22 from the distal end 22d thereof to the proximal end 22p thereof in the longitudinal direction x1. By setting the lower limit value of the ratio of the length L1 of the first balloon 21 to the length L2 of the second balloon 22 to the above range, pressure is easily applied to a sufficient range of the target site by the balloon catheter 1, whereby dilation of the stenosis site and rupture of a bioprosthetic valve are easily performed.

As shown in FIG. 2, the first balloon 21 and the second balloon 22 respectively have straight tubular parts 213, 223, proximal tapered parts 212, 222 located proximal to the straight tubular parts 213, 223, and distal tapered parts 214, 224 located distal to the straight tubular parts 213, 223. That is, the first balloon 21 has a straight tubular part 213, a proximal tapered part 212 located proximal to the straight tubular part 213, and a distal tapered part 214 located distal to the straight tubular part 213, and the second balloon 22 has a straight tubular part 223, a proximal tapered part 222 located proximal to the straight tubular part 223, and a distal tapered part 224 located distal to the straight tubular part 223.

Each of the straight tubular parts 213 and 223 preferably has a substantially columnar shape having a diameter substantially unchanging in the longitudinal direction x1 but may have a diameter varying in the longitudinal direction x1. The proximal tapered parts 212 and 222 and the distal tapered parts 214 and 224 are each preferably formed into a substantially conical shape or truncated conical shape with the diameter thereof being decreased in a direction away from the corresponding straight tubular part 213 or 223. Since the straight tubular parts 213 and 223 have maximum diameters, when the first balloon 21 and the balloon group 30 are inflated in a lesion site such as a stenosis site, the straight tubular parts 223 of the second balloons 22 constituting the balloon group 30 are sufficiently brought into contact with the lesion site, whereby treatment such as dilation of the lesion site can be easily performed. In addition, since the proximal tapered parts 212 and 222 and the distal tapered parts 214 and 224 have such decreased diameters, when the first balloon 21 and the balloon group 30 are deflated, the outer diameters of proximal end parts and distal end parts of the second balloons 22 constituting the balloon group 30 become small, whereby the height differences between the shaft 10 and the balloon group 30 can be made small. Consequently, in a deflated state of the first balloon 21 and the balloon group 30, the outer surface of the balloon catheter 1 becomes smooth, whereby the balloon catheter 1 can be easily inserted into the body cavity.

It is preferable that, in the inflated state of the first balloon 21 and the balloon group 30, a proximal end 212p of the proximal tapered part 212 of the first balloon 21 is located distal to a proximal end 222p of the proximal tapered part 222 of at least one of the second balloons 22, and a distal end 214d of the distal tapered part 214 of the first balloon 21 is located proximal to a distal end 224d of the distal tapered part 224 of the at least one second balloon 22. By the proximal end 212p of the proximal tapered part 212 of the first balloon 21 being located distal to the proximal end 222p of the proximal tapered part 222 of the at least one second balloon 22 and by the distal end 214d of the distal tapered part 214 of the first balloon 21 being located proximal to the distal end 224d of the distal tapered part 224 of the at least one second balloon 22, the position of the proximal tapered part 212 of the first balloon 21 becomes less likely to overlap with the position of the proximal tapered part 222 of the at least one second balloon 22, and the position of the distal tapered part 214 of the first balloon 21 becomes less likely to overlap with the position of the distal tapered part 224 of the at least one second balloon 22. In addition, at the portions of the distal ends 22d and the proximal ends 22p of the plurality of second balloons 22 constituting the balloon group 30, the balloon group 30 is restrained by the shaft 10, and the balloon 2 becomes less likely to be inflated outward of the balloon group 30. As a result, in the inflated state of the balloon 2, the outer diameters of both end portions of the balloon 2 are less likely to become large, and meanwhile, the outer diameter of the center portion of the balloon 2 easily becomes large. Consequently, the outer surface of the balloon group 30 easily comes to have an ellipsoidal shape (so-called rugby ball shape, i.e., prolate spheroidal shape) as a result of being curved into a bow shape about the longitudinal direction x1. Thus, the portion for applying a load through inflation of the balloon 2 is easily set to be the center portion of the balloon 2, and pressure is easily applied only to the target site, whereby the minimal invasiveness can be improved.

Furthermore, by the proximal end 212p of the proximal tapered part 212 of the first balloon 21 being located distal to the proximal end 222p of the proximal tapered part 222 of the at least one second balloon 22 and by the distal end 214d of the distal tapered part 214 of the first balloon 21 being located proximal to the distal end 224d of the distal tapered part 224 of the at least one second balloon 22, the outer diameters of both end portions of the balloon 2 can be made small also in the deflated state of the balloon 2. Thus, the outer diameter of, for example, a sheath to be used for delivering the balloon catheter 1 to the treatment-target site can also be made small, whereby the minimal invasiveness can be further improved.

It is more preferable that, in the inflated state of the first balloon 21 and the balloon group 30, the proximal end 212p of the proximal tapered part 212 of the first balloon 21 is located distal to a distal end 222d of the proximal tapered part 222 of the at least one second balloon 22, and the distal end 214d of the distal tapered part 214 of the first balloon 21 is located proximal to a proximal end 224p of the distal tapered part 224 of the at least one second balloon 22. By the proximal end 212p of the proximal tapered part 212 of the first balloon 21 being located distal to the distal end 222d of the proximal tapered part 222 of the at least one second balloon 22 and by the distal end 214d of the distal tapered part 214 of the first balloon 21 being located proximal to the proximal end 224p of the distal tapered part 224 of the at least one second balloon 22, a configuration is obtained in which: the proximal tapered part 212 of the first balloon 21 does not overlap with the proximal tapered part 222 of the at least one second balloon 22; and the distal tapered part 214 of the first balloon 21 does not overlap with the distal tapered part 224 of the at least one second balloon 22. Thus, the outer diameter of the balloon 2 becomes less likely to increase at the proximal tapered part 222 and the distal tapered part 224 of the at least one of the second balloons 22 constituting the balloon group 30.

As shown in FIG. 2, the first balloon 21 and the second balloon 22 preferably respectively have proximal sleeve parts 211, 221 located proximal to the proximal tapered parts 212, 222, and distal sleeve parts 215, 225 located distal to the distal tapered parts 214, 224. That is, the first balloon 21 preferably has a proximal sleeve part 211 located proximal to the proximal tapered part 212 and a distal sleeve part 215 located distal to the distal tapered part 214, and the second balloon 22 preferably has a proximal sleeve part 221 located proximal to the proximal tapered part 222 and a distal sleeve part 225 located distal to the distal tapered part 224.

It is preferable that: the proximal tapered part 212, the straight tubular part 213, and the distal tapered part 214 of the first balloon 21 are portions to be inflated when a fluid is introduced into the first balloon 21; the proximal tapered part 222, the straight tubular part 223, and the distal tapered part 224 of the second balloon 22 are portions to be inflated when a fluid is introduced into the second balloon 22; and meanwhile, the proximal sleeve parts 211 and 221 and the distal sleeve parts 215 and 225 are non-inflatable portions. By the proximal sleeve parts 211 and 221 and the distal sleeve parts 215 and 225 being not inflatable, a configuration can be obtained in which at least a portion of the proximal sleeve parts 211 and 221 and at least a portion of the distal sleeve parts 215 and 225 are easily fixed to the shaft 10.

A distance in the longitudinal direction x1 from the proximal end 21p of the first balloon 21 to the proximal end 22p of at least one of the second balloons 22 is preferably approximately equal to a distance in the longitudinal direction x1 from the distal end 21d of the first balloon 21 to the distal end 22d of the at least one second balloon 22. That is, the distance in the longitudinal direction x1 from the proximal end 21p of the first balloon 21 to the proximal end 22p of the at least one second balloon 22 is preferably 90% or more and 110% or less of the distance in the longitudinal direction x1 from the distal end 21d of the first balloon 21 to the distal end 22d of the at least one second balloon 22. By the distance from the proximal end 21p of the first balloon 21 to the proximal end 22p of the at least one second balloon 22 being approximately equal to the distance from the distal end 21d of the first balloon 21 to the distal end 22d of the at least one second balloon 22, the first balloon 21 is easily located at the center portion of the balloon group 30 in the longitudinal direction x1. As a result, the portion for applying a load through inflation of the balloon 2 is easily set to be the center portion of the balloon 2, whereby the site to which pressure is to be applied by the balloon 2 is easily adjusted.

The position of a midpoint of the length L1 of the first balloon 21 from the distal end 21d thereof to the proximal end 21p thereof in the longitudinal direction x1 preferably coincides with the position of a midpoint P1 of a length of the balloon group 30 in the longitudinal direction x1. In the longitudinal direction x1, the position of the midpoint of the length L1 of the first balloon 21 from the distal end 21d thereof to the proximal end 21p thereof coinciding with the position of the midpoint P1 of the length of the balloon group 30 makes it easy for the balloon 2 to inflate to a largest extent at the position of the midpoint P1 of the length of the balloon group 30 and thus can make it easy to apply a high pressure at the position of the midpoint P1.

As shown in FIG. 2, in the inflated state of the first balloon 21 and the balloon group 30, an outer diameter of the balloon group 30 at the midpoint P1 of the length of the balloon group 30 in the longitudinal direction x1 is preferably larger than an outer diameter of the balloon group 30 at a distal end 223d of the straight tubular part 223 of the at least one second balloon 22 and an outer diameter of the balloon group 30 at a proximal end 223p of the straight tubular part 223 of the at least one second balloon 22. The outer diameter of the balloon group 30 refers to a diameter of a circumcircle of the plurality of second balloons 22 constituting the balloon group 30. That is, in the inflated state of the balloon 2, the outer diameter of the balloon group 30 at the midpoint P1 of the length of the balloon group 30 is preferably larger than a diameter of a circumcircle of the balloon group 30 at each of the distal end 223d and the proximal end 223p of the straight tubular part 223 of the at least one second balloon 22. When the outer diameter of the balloon group 30 at the midpoint P1 of the length of the balloon group 30 becomes larger than the outer diameter of the balloon group 30 at the distal end 223d of the straight tubular part 223 of the at least one second balloon 22 and the outer diameter of the balloon group 30 at the proximal end 223p of the straight tubular part 223 of the at least one second balloon 22, the balloon group 30 easily comes to have an ellipsoidal outer shape as a result of being curved into a bow shape about the longitudinal direction x1. Thus, at the portion of the midpoint P1 of the length of the balloon group 30, the balloon 2 is inflated to a large extent, whereby pressure is easily applied to the target site. Meanwhile, at the portions of the distal end 223d and the proximal end 223p of the straight tubular part 223 of the at least one second balloon 22, the balloon 2 is less likely to be inflated than at the portion of the midpoint P1 of the length of the balloon group 30. Consequently, pressure becomes less likely to be applied to a site other than the target site, whereby the minimal invasiveness can be improved.

In the inflated state of the first balloon 21 and the balloon group 30, the outer diameter of the balloon group 30 at the midpoint P1 of the length of the balloon group 30 in the longitudinal direction x1 is preferably not less than 1.1 times, more preferably not less than 1.3 times, and further preferably not less than 1.5 times the outer diameter of the balloon group 30 at the distal end 223d of the straight tubular part 223 of the at least one second balloon 22 and the outer diameter of the balloon group 30 at the proximal end 223p of the straight tubular part 223 of the at least one second balloon 22. By setting the lower limit value of the ratio of the outer diameter of the balloon group 30 at the midpoint P1 of the length of the balloon group 30 to each of the outer diameters of the balloon group 30 at the distal end 223d and the proximal end 223p of the straight tubular part 223 of the at least one second balloon 22 to the above range, it becomes easy for inflation to occur such that the outer diameter of the balloon group 30 at the midpoint P1 of the length of the balloon group 30 becomes sufficiently larger than each of the outer diameters of the balloon group 30 at the distal end 223d and the proximal end 223p of the straight tubular part 223 of the at least one second balloon 22. Meanwhile, in the inflated state of the first balloon 21 and the balloon group 30, the outer diameter of the balloon group 30 at the midpoint P1 of the length of the balloon group 30 in the longitudinal direction x1 is preferably not more than 10 times, more preferably not more than 9 times, and further preferably not more than 8 times the outer diameter of the balloon group 30 at the distal end 223d of the straight tubular part 223 of the at least one second balloon 22 and the outer diameter of the balloon group 30 at the proximal end 223p of the straight tubular part 223 of the at least one second balloon 22. By setting the lower limit value of the ratio of the outer diameter of the balloon group 30 at the midpoint P1 of the length of the balloon group 30 to each of the outer diameters of the balloon group 30 at the distal end 223d and the proximal end 223p of the straight tubular part 223 of the at least one second balloon 22 to the above range, the outer diameter of the balloon group 30 becomes less likely to excessively increase at the midpoint P1 of the length of the balloon group 30, whereby the minimal invasiveness of the balloon catheter 1 can be improved.

As shown in FIG. 3, in the inflated state of the first balloon 21 and the balloon group 30, at least one pair of adjacent second balloons 22 among the plurality of second balloons 22 constituting the balloon group 30 are preferably in contact with each other. That is, in the inflated state of the balloon 2, the outer surfaces of at least two of the second balloons 22 are preferably in contact with each other. By at least one pair of adjacent second balloons 22 among the plurality of second balloons 22 constituting the balloon group 30 being in contact with each other, the adjacent second balloons 22 mutually suppress respective inflations when fluids are introduced into both the first balloon 21 and the balloon group 30 to inflate the balloon 2. Thus, the pressure of the fluid introduced into the second balloons 22 in order to inflate the second balloons 22 becomes high, and the hardnesses of the second balloons 22 increase, whereby the expansive force of the balloon 2 can be increased.

In the inflated state of the first balloon 21 and the balloon group 30, all of the second balloons 22 constituting the balloon group 30 are more preferably in contact with second balloons 22 adjacent to the second balloon 22. Specifically, in a case where the balloon 2 of the balloon catheter 1 has a configuration such as one shown in FIG. 3, it is preferable to employ a configuration in which each of the second balloons 22 is in contact with both second balloons 22 located adjacent to the second balloon 22 in the circumferential direction z1. By each of the second balloons 22 constituting the balloon group 30 being in contact with second balloons 22 adjacent to the second balloon 22, all of the second balloons 22 constituting the balloon group 30 mutually suppress respective inflations and the internal pressures of all of the second balloons 22 become high in the inflated state of the balloon 2. Thus, the hardness of the entirety of the balloon group 30 increases, whereby the expansive force of the balloon 2 can be further increased.

As shown in FIG. 3, in the inflated state of the first balloon 21 and the balloon group 30, the balloon group 30 is preferably in contact with an outer peripheral surface of the first balloon 21. That is, in the inflated state of the balloon 2, at least one of the plurality of second balloons 22 constituting the balloon group 30 is preferably in contact with the outer surface of the first balloon 21. By the balloon group 30 being in contact with the outer peripheral surface of the first balloon 21, it becomes easy for the first balloon 21 and the balloon group 30 to mutually suppress respective inflations in the inflated state of the balloon 2. As a result, both the first balloon 21 and the at least one second balloon 22 become less likely to be inflated, and the expansive force of the balloon 2 can be increased. In addition, the first balloon 21 and the balloon group 30 mutually suppressing respective inflations enables exhibition of an advantageous effect of making it less likely for the first balloon 21 and the at least one second balloon 22 to be excessively inflated when fluids are introduced into both the first balloon 21 and the balloon group 30 to obtain high-pressure states.

In the inflated state of the first balloon 21 and the balloon group 30, all of the second balloons constituting the balloon group 30 are more preferably in contact with the outer peripheral surface of the first balloon 21. By all of the second balloons constituting the balloon group 30 being in contact with the outer peripheral surface of the first balloon 21, the advantageous effect for the first balloon 21 and the balloon group 30 to mutually suppress respective inflations is easily improved, whereby it becomes easy to further increase the expansive force of the balloon 2.

As shown in FIG. 1, FIG. 4, and FIG. 5, it is preferable that: the shaft 10 has a first inflation lumen 11 and a second inflation lumen 12 extending in the longitudinal direction x1; the first inflation lumen 11 is connected to the first balloon 21; and the second inflation lumen 12 is connected to the at least one of the second balloons 22 constituting the balloon group 30. By the shaft 10 having the first inflation lumen 11 connected to the first balloon 21 and the second inflation lumen 12 connected to the at least one second balloon 22, a configuration is obtained in which the first balloon 21 and the at least one second balloon 22 are connected to the respective different inflation lumens. Thus, the inflation timing and the inflation pressure can be set to differ between the first balloon 21 and the balloon group 30 including the plurality of second balloons 22, whereby the balloon catheter 1 can be set to be capable of adaptation to various treatments, various situations regarding lesion sites, and the like.

In addition, by the shaft 10 having the first inflation lumen 11 connected to the first balloon 21 and the second inflation lumen 12 connected to the at least one second balloon 22, the fluid inside the first balloon 21 can be discharged through the first inflation lumen 11 after the balloon 2 of the balloon catheter 1 is disposed in a target site of a lumen inside a living organism such as a blood vessel and the first balloon 21 and the balloon group 30 are inflated. As a result, the first balloon 21 can be deflated to increase the perfusion rate of blood in the state where the balloon 2 is disposed inside the blood vessel. The increase in the perfusion rate of blood can lead to exhibition of: an advantageous effect of eliminating the need for temporary stoppage of the heart (so-called rapid pacing) in a treatment for aortic valve stenosis or the like so that the treatment can be performed in a minimally invasive manner; and an advantageous effect of enabling suppression of displacement of the balloon 2 by passage of the blood during inflation of the balloon 2 so that it can be made less likely to damage a site other than the target site such as a lesion site.

FIG. 1 shows a so-called rapid exchange type balloon catheter 1 having a guide wire tube 40, which has a guide wire port 50 located midway between the distal and proximal sides of the shaft 10 and functions as a guide wire insertion path extending from the guide wire port 50 to the distal side of the shaft 10. The balloon catheter 1 preferably has a distal shaft 15 and a proximal shaft 16. The distal shaft 15 and the proximal shaft 16 may be separate members, and a proximal end part of the distal shaft 15 may be connected to a distal end part of the proximal shaft 16 to form a shaft 10 extending from the balloon 2 to a proximal end part of the balloon catheter 1. Alternatively, one shaft 10 may extend from the balloon 2 to the proximal end part of the balloon catheter 1. Alternatively, the distal shaft 15 and the proximal shaft 16 may be further composed of a plurality of tube members.

The shaft 10 preferably has therein a fluid flow path and a guide wire insertion path. Examples of the configuration in which the shaft 10 has therein a fluid flow path and a guide wire insertion path include a configuration in which: the guide wire tube 40 disposed inside the shaft 10 functions as the guide wire insertion path; and a space between the shaft 10 and the guide wire tube 40 functions as the fluid flow path. In the case of such a configuration, it is preferable that: the guide wire tube 40 extends outward from the distal end of the shaft 10 and penetrates the balloon 2; the distal side of the balloon 2 is connected to the guide wire tube 40; and the proximal side of the balloon 2 is connected to the shaft 10.

The shaft 10 is preferably composed of resin, metal, or a combination of resin and metal. By using resin as the material for the shaft, flexibility and elasticity can be more easily imparted to the shaft 10. By using metal as the material for the shaft 10, the delivering performance of the balloon catheter 1 can be improved. Examples of resin used for the shaft 10 include polyamide-based resin, polyester-based resin, polyurethane-based resin, polyolefin-based resin, fluorine-based resin, polyvinyl chloride-based resin, silicone-based resin, natural rubber, and synthetic rubber. Any one of these may be used alone, or two or more may be used in combination. Examples of metal used for the shaft 10, include stainless steel such as SUS 304 and SUS 316, platinum, nickel, cobalt, chromium, titanium, tungsten, gold, Ni-Ti alloys, Co-Cr alloys, or combinations thereof. When the shaft 10 is composed of the distal shaft 15 and proximal shaft 16 as separate members, the distal shaft 15 may be, for example, made of resin, and the proximal shaft 16 may be made of metal. The shaft 10 may also have a layered structure using different materials or the same material.

As shown in FIG. 1 to FIG. 5, it is preferable that: the shaft 10 has a guide wire lumen 13 extending in the longitudinal direction x1 and through which a guide wire is inserted; the balloon catheter 1 further has a guide wire tube 40 having a lumen in communication with the guide wire lumen 13; and the guide wire tube 40 is disposed in the lumen of the first balloon 21. By the balloon catheter 1 having the guide wire tube 40 having the lumen in communication with the guide wire lumen 13, a guide wire is easily inserted into the balloon catheter 1, whereby the balloon catheter 1 can be delivered into the body along the guide wire. In addition, by inserting a guide wire into the guide wire tube 40, the guide wire can be prevented from damaging the first balloon 21, the second balloons 22, or the like.

Examples of a material forming the guide wire tube 40 include synthetic resins such as: polyolefin-based resins such as polyethylene and polypropylene; polyamide-based resins such as nylon; polyester-based resins such as PET; aromatic polyether ketone-based resins such as PEEK; polyether polyamide-based resins; polyurethane-based resins; polyimide-based resins; fluorine-based resins such as PTFE, PFA, and ETFE; and polyvinyl chloride-based resins. Among these materials, a polyimide-based resin is preferable as the material forming the guide wire tube 40. By the material forming the guide wire tube 40 being a polyimide-based resin, the slipperiness of the guide wire tube 40 is improved. Thus, it becomes easy to insert a guide wire into the lumen of the guide wire tube 40 and deliver the balloon catheter 1 into the body along the guide wire. The guide wire tube 40 may have a multilayer structure including a braid layer of a metal braid or the like. By the guide wire tube 40 having such a multilayer structure, the strength, the slipperiness with respect to a guide wire, and kink-proof properties of the guide wire tube 40 can be increased.

As shown in FIG. 1, the guide wire tube 40 preferably has a proximal end part connected to a distal end part of the shaft 10. In the case of a configuration in which the shaft 10 has the distal shaft 15 and the proximal shaft 16, the proximal end part of the guide wire tube 40 is preferably connected to a distal end part of the distal shaft 15. By the proximal end part of the guide wire tube 40 being connected to the distal end part of the shaft 10, the outer diameter of the balloon catheter 1 is less likely to become large, whereby the minimal invasiveness can be improved.

The balloon 2 and the shaft 10 may be joined by adhesive bonding, welding, or by attaching a ring-shaped member at the point where the end of the balloon 2 and the shaft 10 overlap to swage them. Of these, the balloon 2 and the shaft 10 are preferably joined by welding. By welding the balloon 2 and the shaft 10, the bond between the balloon 2 and the shaft 10 is difficult to be released even when the balloon 2 is repeatedly inflated and deflated, easily increasing the strength of the bond between them.

The balloon catheter 1 is preferably provided with a tip member 60 at its distal end part. The tip member 60 may be provided at the distal end part of the balloon 2 by being connected to the distal end part of the balloon 2 as a separate component from the guide wire tube 40, or the guide wire tube 40 extending distally beyond the distal end of the balloon 2 may function as the tip member 60.

As shown in FIG. 1 and FIG. 2, the guide wire tube 40 inside the balloon 2 has a portion corresponding to the position of the balloon 2 in the longitudinal direction x1, and, on this portion, a radiopaque marker 70 may be disposed such that the position of the balloon 2 can be ascertained radiographically.

Examples of the position on the guide wire tube 40 at which the radiopaque marker 70 is disposed include: the position of the midpoint of the length L1 of the first balloon 21 from the distal end 21d thereof to the proximal end 21p thereof; each of the positions of a proximal end 213p and a distal end 213d of the straight tubular part 213 of the first balloon 21; and each of the positions of the proximal end 223p and the distal end 223d of the straight tubular part 223 of at least one of the second balloons 22. Among these positions, each of the positions of the proximal end 213p and the distal end 213d of the straight tubular part 213 of the first balloon 21 is preferable as the position on the guide wire tube 40 at which the radiopaque marker 70 is disposed. By the radiopaque marker 70 being disposed on the guide wire tube 40 at each of the positions of the proximal end 213p and the distal end 213d of the straight tubular part 213 of the first balloon 21, the position at which the balloon 2 is to be inflated to a large extent by the first balloon 21 is easily ascertained. As a result, the balloon catheter 1 can be set to easily apply pressure to the target site.

As shown in FIG. 1, a hub 5 may be provided at a proximal side of the shaft 10. The hub 5 may be further provided with a fluid inlet 6 that is communicated with the flow path of the fluid supplied to the interior of the balloon 2.

The shaft 10 and the hub 5 may be joined by, for example, adhesive bonding or welding. Of these, the shaft 10 and the hub 5 are preferably joined by adhesive bonding. The adhesive bonding of the shaft 10 and the hub 5 can increase the bonding strength of the shaft 10 and hub 5 to increase durability of the balloon catheter 1 when the materials forming the shaft 10 and hub 5 are different, for example, in a case where the shaft 10 is made of material having high flexibility and the hub 5 is made of material having high stiffness.

Although not shown in the figures, the present invention is also applicable to a so-called over-the-wire type balloon catheter that has a guidewire lumen extending from the distal side to the proximal side of the shaft. In the case of the over-the-wire type, the inflation lumen and the guidewire lumen preferably extend to a hub positioned at the proximal side, and the proximal openings of each lumen are preferably provided in the hub having a bifurcated structure.

As shown in FIG. 1, in the case of the rapid-exchange type catheter, the outer wall of the distal shaft 15 and/or the proximal shaft 16 is preferably coated as appropriate, and more preferably, both the distal shaft 15 and the proximal shaft 16 are coated. In the case of the over-the-wire type catheter, the outer wall of the outer shaft is preferably coated as appropriate.

The coating applied to the shaft 10 can be a hydrophilic or hydrophobic coating, depending on the purpose, and can be applied by dipping the shaft 10 into a hydrophilic or hydrophobic coating agent, applying a hydrophilic or hydrophobic coating agent to the outer wall of the shaft 10, or coating the outer wall of the shaft 10 with a hydrophilic or hydrophobic coating agent. The coating agent may contain medical agents and additives.

Hydrophilic coating agents include hydrophilic polymers such as polyvinyl alcohol, polyethylene glycol, polyacrylamide, polyvinyl pyrrolidone, methyl vinyl ether maleic anhydride copolymer, and hydrophilic coating agents made of any combination thereof.

Hydrophobic coating agents include polytetrafluoroethylene (PTFE), ethylenepropylene fluoride (FEP), perfluoroalkoxy alkane (PFA), silicone oil, hydrophobic urethane resin, carbon coat, diamond coat, diamond-like carbon (DLC) coating, ceramic coating, and substances with low surface free energy terminated with an alkyl group or a perfluoroalkyl group.

The balloon catheter 1 of the present invention is preferably used for dilation of an aortic valve, deformation of a bioprosthetic valve implanted in a heart, or rupture of the bioprosthetic valve. Specifically, the balloon catheter 1 of the present invention is preferably used for the purpose of: dilating an aortic valve hardened by calcification and other factors; or deforming or rupturing, for example, an annuloplasty ring for a bioprosthetic valve having been implanted in a heart but degraded, in order to replace the bioprosthetic valve. The balloon catheter 1 of the present invention easily applies a high pressure at the portion thereof at which the first balloon 21 is present and thus easily achieves deformation or rupture of a bioprosthetic valve or dilation of a hardened aortic valve that has been unable to be sufficiently dilated by a conventional balloon catheter. Therefore, the balloon catheter 1 can be suitably used.

The present application claims priority based on Japanese Patent Application No. 2023-102816 filed on June 22, 2023. All the contents described in Japanese Patent Application No. 2023-102816 filed on June 22, 2023 are incorporated herein by reference.

### DESCRIPTION OF REFERENCE SIGNS

1: balloon catheter
2: balloon
5: hub
6: fluid inlet
10: shaft
11: first inflation lumen
12: second inflation lumen
13: guide wire lumen
15: distal shaft
16: proximal shaft
21: first balloon
21d: distal end of the first balloon
21p: proximal end of the first balloon
211: proximal sleeve part of the first balloon
211 d: distal end of the proximal sleeve part of the first balloon
211p: proximal end of the proximal sleeve part of the first balloon
212: proximal tapered part of the first balloon
212d: distal end of the proximal tapered part of the first balloon
212p: proximal end of the proximal tapered part of the first balloon
213: straight tubular part of the first balloon
213d: distal end of the straight tubular part of the first balloon
213p: proximal end of the straight tubular part of the first balloon
214: distal tapered part of the first balloon
214d: distal end of the distal tapered part of the first balloon
214p: proximal end of the distal tapered part of the first balloon
215: distal sleeve part of the first balloon
215d: distal end of the distal sleeve part of the first balloon
215p: proximal end of the distal sleeve part of the first balloon
22: second balloon
22d: distal end of the second balloon
22p: proximal end of the second balloon
221: proximal sleeve part of the second balloon
221 d: distal end of the proximal sleeve part of the second balloon
221p: proximal end of the proximal sleeve part of the second balloon
222: proximal tapered part of the second balloon
222d: distal end of the proximal tapered part of the second balloon
222p: proximal end of the proximal tapered part of the second balloon
223: straight tubular part of the second balloon
223d: distal end of the straight tubular part of the second balloon
223p: proximal end of the straight tubular part of the second balloon
224: distal tapered part of the second balloon
224d: distal end of the distal tapered part of the second balloon
224p: proximal end of the distal tapered part of the second balloon
225: distal sleeve part of the second balloon
225d: distal end of the distal sleeve part of the second balloon
225p: proximal end of the distal sleeve part of the second balloon
30: balloon group
40: guide wire tube
50: guide wire port
60: tip member
70: radiopaque marker
L1: length from the distal end of the first balloon to the proximal end of the first balloon
L2: length from the distal end of the second balloon to the proximal end of the second balloon
P1: midpoint of the length of the balloon group

## Claims

1. A balloon catheter comprising:
a shaft extending in a longitudinal direction from a proximal side to a distal side;
a first balloon disposed at a distal part of the shaft; and
a balloon group including a plurality of second balloons disposed outside the first balloon and arranged in a circumferential direction of the first balloon, wherein
in an inflated state of the first balloon and the balloon group, a length of the first balloon from a distal end thereof to a proximal end thereof in the longitudinal direction is shorter than a length of at least one of the plurality of second balloons from a distal end thereof to a proximal end thereof in the longitudinal direction.

2. The balloon catheter according to claim 1, wherein
the first balloon and the at least one of the plurality of second balloons each have a straight tubular part, a proximal tapered part located proximal to the straight tubular part, and a distal tapered part located distal to the straight tubular part, and
in the inflated state of the first balloon and the balloon group, a proximal end of the proximal tapered part of the first balloon is located distal to a proximal end of the proximal tapered part of the at least one of the plurality of second balloons, and a distal end of the distal tapered part of the first balloon is located proximal to a distal end of the distal tapered part of the at least one of the plurality of second balloons.

3. The balloon catheter according to claim 2, wherein
in the inflated state of the first balloon and the balloon group, an outer diameter of the balloon group at a midpoint of a length of the balloon group in the longitudinal direction is larger than an outer diameter of the balloon group at a distal end of the straight tubular part of the at least one of the plurality of second balloons and an outer diameter of the balloon group at a proximal end of the straight tubular part of the at least one of the plurality of second balloons.

4. The balloon catheter according to claim 1, wherein
in the inflated state of the first balloon and the balloon group, at least one pair of adjacent second balloons among the plurality of second balloons constituting the balloon group are in contact with each other.

5. The balloon catheter according to claim 1, wherein
in the inflated state of the first balloon and the balloon group, the balloon group is in contact with an outer peripheral surface of the first balloon.

6. The balloon catheter according to claim 1, wherein
the shaft has a first inflation lumen and a second inflation lumen extending in the longitudinal direction,
the first inflation lumen is connected to the first balloon, and
the second inflation lumen is connected to the at least one of the plurality of second balloons constituting the balloon group.

7. The balloon catheter according to claim 1, wherein
the shaft has a guide wire lumen extending in the longitudinal direction and through which a guide wire is inserted,
the balloon catheter further comprises a guide wire tube having a lumen in communication with the guide wire lumen, and
the guide wire tube is disposed in a lumen of the first balloon.

8. The balloon catheter according to any one of claims 1 to 7, wherein
the balloon catheter is used for dilation of an aortic valve, deformation of a bioprosthetic valve implanted in a heart, or rupture of the bioprosthetic valve.
